(19) Europäisches Patentamt
European Patent Office
Office européen des brevets

(11) Publication number : 0 197 657 B1

(12) EUROPEAN PATENT SPECIFICATION

(45) Date of publication of patent specification : 27.02.91 Bulletin 91/09

(51) Int. Cl.$^5$ : C07C 231/06, C07D 295/18, C08G 69/08

(21) Application number : 86301647.3

(22) Date of filing : 07.03.86

# (54) A novel process for preparing amides from nitriles and amines.

(30) Priority : 08.03.85 JP 44939/85

(43) Date of publication of application : 15.10.86 Bulletin 86/42

(45) Publication of the grant of the patent : 27.02.91 Bulletin 91/09

(84) Designated Contracting States : DE GB

(56) References cited :
EP-A- 0 070 424
US-A- 3 825 596
US-A- 3 948 989
US-A- 4 060 553
US-A- 4 380 623

(73) Proprietor : OSAKA UNIVERSITY
1-1 Yamadaoka
Suita City Osaka-Fu (JP)

(72) Inventor : Murahashi, Shunichi
3-29, Asahigaoka 1-Chome
Ikeda City Osaka-Fu (JP)
Inventor : Naota, Takeshi
5-9 Midoridai 3-Chome
Kawanishi City Hyogo Pref. (JP)

(74) Representative : Lambert, Hugh Richmond et al
D. YOUNG & CO. 10 Staple Inn
London, WC1V 7RD (GB)

EP 0 197 657 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to the preparation of amides from nitriles and amines of polyamides directly from dinitriles and diamines or from aminonitriles.

Many kinds of amide compounds including polyamides have so far been developed and used in various industrial fields. These are particularly, but not exclusively, useful as materials for synthetic fibres and plastics moulds, dyeability improvers or antistatic agents for such items, finishing or sizing agents for yarns and textiles, surfactants, coagulants, adhesives, organic solvents, plastics-foaming agents, printing-ink additives, dyestuffs, organic pigments, pharmaceuticals, agricultural chemicals, livestock feeds, and intermediate compounds for preparing such materials.

Hitherto, when an amide has been synthesized from a nitrile and an amine, usually the nitrile is hydrolysed and thereby converted into carboxylic acid which is subsequently condensed with the amine.

Two US Patent Specifications and one European Patent Specification are particular examples of the synthesis of amides from a nitrile and an amine in the presence of water.

Thus US-A-3 825 596 discloses a process for preparing N,N-dialkyl toluamide by reacting tolunitrile with a di- or trialkylamine and water under heated, preferably pressurised conditions, optionally in the presence of a catalyst. Suggested and tried catalysts include zinc chloride, mercuric chloride, cadmium acetate, lead acetate, zinc acetate, copper acetate, cobalt acetate and nickel acetate.

US-A-4 380 623 discloses a process for preparing amides comprising reacting a nitrile, an amine and water in the presence of a carbon dioxide catalyst. In particular, the preparation of nylon-6,6 is described by reacting adiponitrile, hexamethylene diamine and water in the presence of $CO_2$.

EP-A-0 070 424 discloses a process for preparing amides from nitriles and amines in the presence of water and a catalyst at a temperature between 80 and 220°C. The catalyst in this case is a copper derivative which may also contain chromium, molybdenum, vanadium, manganese or zinc.

However, conventional manufacturing processes result in various problems. These are related inter alia to increase of process steps, separation of products in each step, equipment for preventing pollution caused by by-products, and the cost of production.

The present invention provides a process for the preparation of organic amides by reaction of a carbonitrile and a primary or secondary amine in which the carbonitrile is reacted directly with an equivalent amount of the primary or secondary amine, the reaction taking place by heating the reactants in the presence of at least a stoichiometric amount of water relative to the carbonitrile reactant, and in the presence of a catalyst comprising a complex containing one or more of ruthenium, rhodium, and molybdenum. This process has a minimum number of steps, so that reaction time is shorter, equipment more compact and operation cleaner.

In one of its embodiments the invention develops the abovementioned direct synthesis of amides into synthesis of polyamides with similar advantages.

In this specification, the term "carbonitrile" or"nitrile" means an organic compound having at least one cyano group in its molecule, and the term "amine" an organic compound having at least one amino group. Both the cyano group and the amino group may be contained in one molecule, in which case they constitute an aminonitrile compound. Further, the term "amide" means an organic compound having at least one amide linkage in its molecule, including a so-called polyamide.

The present invention includes the following three principal and preferred embodiments.

In the first preferred embodiment of the invention, the nitrile is represented by the general formula $R^1CN$ where $R^1$ denotes (i) an alkyl, alkenyl, alkynyl, cycloalkyl or aryl group of up to 20 carbon atoms, or (ii) a univalent residue of a 3 to 7 membered heterocyclic group having in the ring up to 3 hetero atoms each of which is O, N or S, with the proviso that the groups defined for $R^1$ optionally contain one or more of the following substituents : an aryl, alkenyl or alkynyl group of up to 12 carbon atoms, a univalent residue of a 3 to 7 membered heterocyclic group containing up to 3 hetero atoms each of which is O, N or S, or an OR, $CO_2R$, $NR_2$, SR, $SiR_3$ or $CONR_2$ group where R is an optionally substituted alkyl group containing up to 10 carbon atoms or a phenyl group ; and the amine reactant is of the formula $R^2R^3NH$ where each of $R^2$ and $R^3$ represents a hydrogen atom or a group as defined for $R^1$, or $-NR^2R^3$ is a saturated or unsaturated heterocyclic ring optionally containing O, S or another N as hetero atom. In such a case the amide product may be represented by the formula $R^1{-}CO{-}NR^2R^3$ where $R^1$, $R^2$ and $R^3$ are as defined above.

In the second principal embodiment of the invention, the carbonitrile reactant is a dinitrile of the formula $R^4(CN)_2$ where $R^4$ denotes (i) an alkylene, alkenylene, alkynylene, cycloalkylene or arylene group having up to 20 carbon atoms ; (ii) a bivalent residue of a 3 to 7 membered heterocyclic group having in the ring up to 3 hetero atoms each of which is O, N or S ; or (iii) a group consisting of two aliphatic hydrocarbon residues each having up to 10 carbon atoms bridged by phenylene group, an O, N or S hetero atom, or a

bivalent residue of a 3 to 7 membered heterocyclic group having in the ring up to 3 hetero atoms each of which is O, N or S ; with the proviso that any of such groups optionally contain one or more of the following substituents : an aryl, alkenyl or alkynyl group of up to 12 carbon atoms, a univalent residue of a 3 to 7 membered heterocyclic group containing up to 3 hetero atoms each of which is O, N or S, or an OR, $CO_2R$, $NR_2$, SR, $SiR_3$ or $CONR_2$ group where R is an optionally substituted alkyl group containing up to 10 carbon atoms or a phenyl group ; and the amine is a diamine of the formula $HN(R^5) - R^6 - (R^7)NH$ where each of $R^5$ and $R^7$ represents a hydrogen atom or a group as defined for $R^1$, and $R^6$ is a group as defined for $R^4$.

In this case the product is a polyamide having a recurring unit represented by the following general formula :

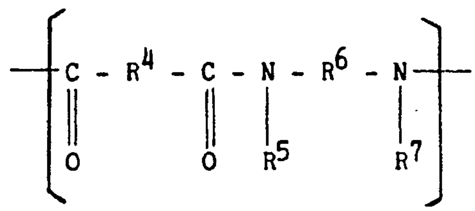

Finally, in the third embodiment of the invention, both the carbonitrile and the amine reactant are the same or different aminonitriles of the formula $HN(R^5)–R^6–CN$ where $R^5$ and $R^6$ are as defined as above. In this case the product is a polyamide having recurring units of the formula

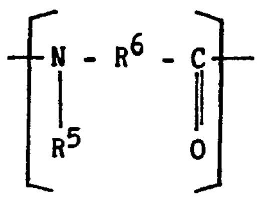

Depending upon whether the same or two different aminonitriles are used as the nitrile and amine reactants, the repeating units in the polyamide may or may not be the same.

Examples of nitriles for use in the first embodiment include acetonitrile, propionitrile, butyronitrile, acrylonitrile, methacrylonitrile, 2-methyl-2-butenenitrile, 2-pentenenitrile, 3-pentenenitrile, cinnamonitrile, cyclohexanecarbonitrile, benzonitrile, 2-thiazolecarbonitrile and methoxyacetonitrile. Further, when the reacting amine is a monoamine, nitriles having two or more cyano groups, such as 1,4-dicyanobutane, 1,6-dicyclohexane and methylglutaronitrile may be included in the first embodiment.

Examples of amines for use in the first embodiment include methylamine, ethylamine, butylamine, diethylamine, benzylamine, benzylmethylamine, cyclohexylamine, aniline, 2-benzofuranamine, anisidine, pyrrolidine, piperidine and morpholine. Further, when the reacting nitrile is a mononitrile, amines having two or more amine groups, e.g. hexamethylenediamine, 1,2-diaminocyclohexane, diaminopiperazines, and bis-hexamethylenetriamines may be used in the first embodiment.

In the second embodiment, preferably dinitriles are alkylenedinitriles having the general formula $NC(CH_2)_nCN$ where n is an integer of 1-20, while preferable diamines are alkylenediamines having the general formula $HN(R^5)–(CH_2)m–NHR^7$ where m is an integer of 1-20, and from those reactants are obtained polyamides having a recurring unit represented by the general formula :

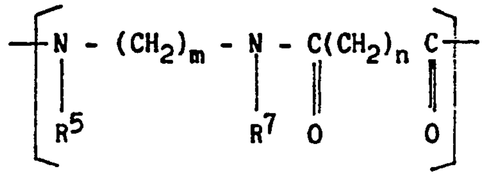

where $R^5$ and $R^7$ are as defined above.

In the third embodiment, preferable aminonitriles are aminoalkylnitriles having the general formula $NC(CH_2)_pNHR^5$ where p is an integer of 1-3 or 5-20, from which will result polyamides having a recurring unit represented by the general formula :

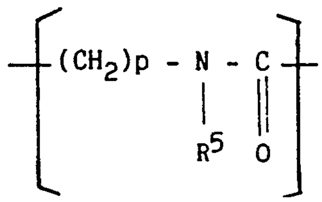

where $R^5$ is as defined above.

In the second and third embodiments, two or more dinitriles and/or diamines and two or more aminonitriles may be used and in these cases copolyamides will be obtained. Moreover, nitriles and/or amines and aminonitriles that are trifunctional or polyfunctional also can be added in such a manner that nearly stoichiometric proportions as a whole are attained, if required, in order to improve the properties of the resulting polyamides.

A small amount of monoamine, mononitrile or monocarboxylic acid may be added to the reaction mixture as a polymerization inhibitor in the second and third embodiments of the invention, in order to control the polymer viscosity.

Catalysts preferred in the process of the present invention are the ruthenium complexes $RuH_2(PPh_3)_4$ and $RuH_2(CO)(PPh_2)_3$, the molybdenum complex $Mo(CO)_6$ and the rhodium complex $Rh(CO)(OH)(PPh_3)$, which have high activity.

These catalysts may be used alone or in combination and, if required, along with an appropriate promoter such as a metal hydroxide.

Only a catalytic amount is normally required, e.g. 0.001 to 10 mol %, preferably 0.1 to 3 mol %, based on the starting nitrile, although the reaction can be effected with smaller or larger amounts.

It is preferred for the reaction to be carried out in an inert gas. Although the reaction readily proceeds on addition of only the catalyst to amines, nitriles (or aminonitriles) and water, it will be carried out more effectively in the presence of a water-miscible organic solvent such as 1,2-dimethoxyethane, dioxane, pyridine, diglyme or tetrahydrofuran. Though the reaction temperature has no specified upper limit, not higher than 250°C is preferable. The reaction pressure may be atmospheric or higher, if required. The amount of water required is at least one equivalent relative to the nitrile reactant. In general, however, a stoichiometric excess of water will be present, e.g. in the range 1-100 equivalents of water, preferably 1-3 equivalents, relative to the nitrile.

Some of the preferred embodiments of the present invention will be illustrated by way of the following examples.

Example 1

Synthesis of N-butylacetamide

A magnetic stirrer was put into a test tube of 30-ml capacity, and argon gas was admitted to displace the air. Acetonitrile (2.0 mmol), butylamine (2.2 mmol), water (4.0 mmol), $RuH_2(PPh_3)_4$ (0.06 mmol) and 1,2-dimethoxyethane (DME, 0.5 ml) were placed in the test tube, which was thereafter sealed.

The solution was allowed to react to 160°C for 24 hours while stirring. After cooling to −78°C, the sealed tube was opened and the product was isolated by passing through a short Florisil (Registered Trade Mark) column. N-butylacetamide was obtained in a 93% yield. Identification of N-butylacetamide was conducted by means IR, NMR and mass spectrum data.

Examples 2-23

Examples in which reaction was carried out under the same conditions as in Example 1 are shown in Table 1.

Table 1(a)

| Example No. | Nitrile | Amine | Product *3 | Yield *4 (%) |
|---|---|---|---|---|
| 2 | $CH_3CN$ | $BuNH_2$ *1 | 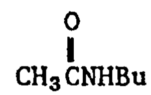 *1 | 93 |
| 3 | $CH_3CN$ | 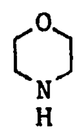 | 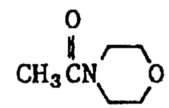 | 99 |
| 4 | $CH_3CN$ | 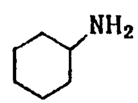 | 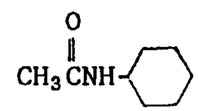 | 84 |
| 5 | $CH_3CN$ | $PhCH_2NH_2$ | 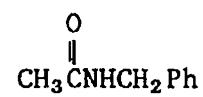 | 98 |
| 6 | $CH_3CN$ | $PhCH_2NHCH_3$ | 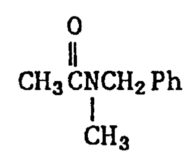 | 95 |

Table 1(b)

| Example No. | Nitrile | Amine | Product *3 | Yield *4 (%) |
|---|---|---|---|---|
| 7 | $CH_3CN$ | $PhNH_2$ | $CH_3\overset{O}{\overset{\|}{C}}NHPh$ | 55 |
| 8 | $CH_3CN$ *2 | $H_2N(CH_2)_6NH_2$ | $CH_3\overset{O}{\overset{\|}{C}}NH(CH_2)_6NH\overset{O}{\overset{\|}{C}}CH_3$ | 89 |
| 9 | $C_3H_7CN$ | 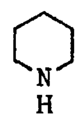 | 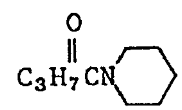 | 90 |
| 10 | $CH_3OCH_2CN$ | $BuNH_2$ | $CH_3OCH_2\overset{O}{\overset{\|}{C}}NHBu$ | 93 |
| 11 | $CH_3OCH_2CN$ | 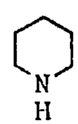 | 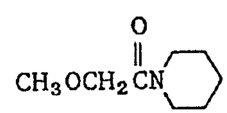 | 87 |

Table 1(c)

| Example No. | Nitrile | Amine | Product *3 | Yield *4 (%) |
|---|---|---|---|---|
| 12 | PhCH=CHCN | 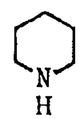 | 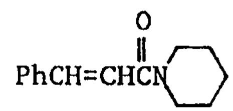 | 57 |
| 13 | PhCN | 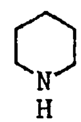 | 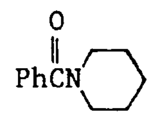 | 50 |
| 14 | $NC(CH_2)_2CN$ | 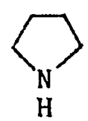 *2 | 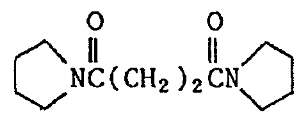 | 91 |
| 15 | $CH_3CN$ | $Bu_2NH$ | 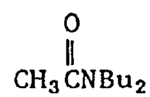 | 61 |

Table 1(d)

| Example No. | Nitrile | Amine | Product *3 | Yield *4 (%) |
|---|---|---|---|---|
| 16 | $CH_3CN$ | 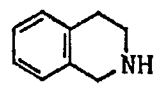 | 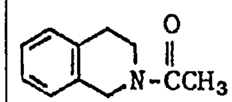 | 59 |
| 17 | $CH_3CN$ *5 | $H_2N(CH_2)_4NH(CH_2)_3NH_2$ | 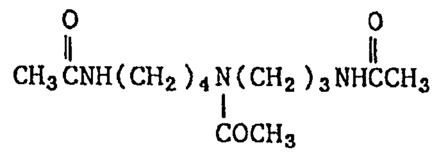 | 75 |
| 18 | 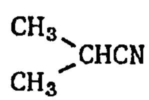 | 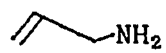 | 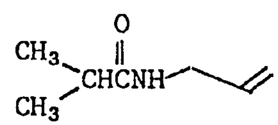 | 32 |
| 19 | 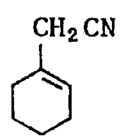 | $CH_3OCH_2CH_2NH_2$ | 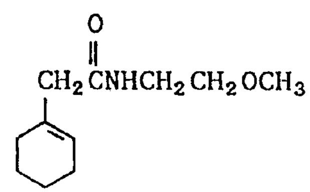 | 54 |

Table 1(e)

| Example No. | Nitrile | Amine | Product *3 | Yield *4 (%) |
|---|---|---|---|---|
| 20 | 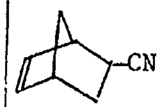 | 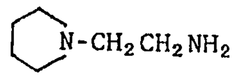 | 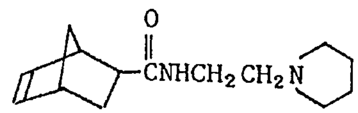 | 41 |
| 21 | 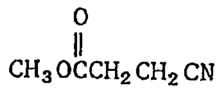 | 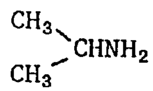 | 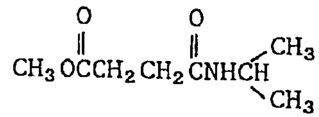 | 38 |
| 22 | PhCN | $BuNH_2$ | 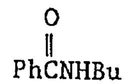 | 30 |
| 23 | 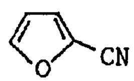 | 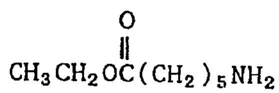 | 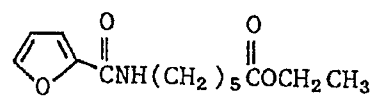 | 32 |

*1 ... Bu : butyl group.
*2 ... 2 equivalent.
*3 ... Each product was identified by IR< NMR and mass spectrum data.
*4 ... Isolated yield.
*5 ... 5 equivalent.

Although a ruthenium compound was used as the catalyst in Examples enumerated above, it was confirmed that amides were also obtainable using rhodium and molybdenum complexes in lieu of the ruthenium compound.

Examples 24-45

The catalyst $RuH_2(PPh_3)_4$ used in the reaction in Example 1 was replaced by the under-mentioned 22 compounds and respective series of the reaction were carried out to obtain N-butylacetamide in yields and with the acetonitrile conversions respectively given in Table 2 below. It will be seen that catalysts in accordance with the invention (Examples 24 to 27 and 31) are superior in yield and/or conversion to most of the comparative Examples 28 to 30 and 32 to 45.

Table 2

| Example No. | Catalyst | Conversion (%) | Yield[a] (%) |
|---|---|---|---|
| 24 | $RuH_2(PPh_3)_4$ | 100 | 99[b] |
| 25 | $RuH_2(CO)(PPh_3)_3$ | 100 | 100 |
| 26 | $[Ru(NH_3)_5C\ell]C\ell_2$ | 54 | 90 |
| 27 | $Rh(CO)(OH)(PPh_3)_2$ | 41 | 51 |
| 28 | Ni(piaH)*1$_2 \cdot C\ell_2 \cdot 2H_2O$ | 43 | 23 |
| 29 | $PdC\ell_2$ | 39 | 42 |
| 30 | $Fe(CO)_5$ | 17 | 15 |
| 31 | $Mo(CO)_6$ | 66 | 77 |
| 32 | Cu(O) | 30 | 33 |
| 33 | $Al(OH)_2(C_{17}H_{35}CO_2)$ | 8 | 25 |
| 34 | $Ti(Oi\text{-}Pr)_4$ | 17 | 99 |
| 35 | $VO(AcAc)_2$ | 60 | 5 |
| 36 | $Cr(CO)_6$ | 9 | 99 |
| 37 | $CoSO_4 \cdot 7H_2O$ | 34 | 12 |
| 38 | $ZnCl_2$ | 76 | 99 |
| 39 | $SeO_2$ | 13 | 52 |
| 40 | $ZrCl_2Cp_2$ | 21 | 98 |
| 41 | $CdCl_2$ | 52 | 20 |
| 42 | $IrCl_3$ | 77 | 10 |
| 43 | $PtO_2$ | 20 | 3 |
| 44 | $HgCl_2$ | 34 | 36 |
| 45 | $Pb(OAc)_4$ | 36 | 49 |

*1 : Picolinic acid amide

a : GLC yield based on acetonitrile

b : Isolated yield.

Example 46

Synthesis of nylon-66

Adiponitrile (0.216 g), hexamethylenediamine (0.232 g), $RuH_2(PPh_3)_4$ (0.069 g), water (0.072 g) and 1,2-dimethoxyethane (0.5 mℓ) were reacted in an argon gas atmosphere in a sealed tube under the same conditions as Example 1. After the reaction, precipitates were separated by filtration, washed with chloroform and dried. Then, nylon-66 was obtained in a 92% yield. Its number average molecular weight was 4,100 which was calculated from terminal amino-groups quantified by p-toluene sulfonic acid using thymol blue as an indicator The nylon-66 was identified by IR (KBr) spectrum which showed absorptions at 3,230 (N–H, m), 2,910 (C–H, s), 2,840 (s), 1,630 (C=O, s), 1,530 (N–H, s), 1,225 (w) and 740 (w) $cm^{-1}$; and by 'HNMR spectrum ($HCO_2H$, 60MHz) : δ 0.93-1.85 (m, 12H, $-CH_2-$), 1.95-2.60 (m, 4H, $-COH_2-$), 2,82-3.43 (m, 4H, $-N-CH_2-$) and 8.45 (br.s, 2H, –NH).

Example 47

Synthesis of high molecular weight nylon-66

Adiponitrile (0.216 g), hexamethylenediamine (0.232 g), $RuH_2(PPh_3)_4$ (0.069 g), water (0.072 g) and 1,2-dimethoxyethane (0.5 mℓ) were reacted at 200°C in an argon gas atmosphere for 24 hours in a sealed tube. After the reaction, precipitates were separated by filtration, washed with chloroform and dried. Then, nylon-66 having a melting point temperature higher than 255°C was obtained in a 98% yield. Its infrared and 'H NMR spectra similar to those in Example 46 resulted. The number average molecular weight was 8,900 which was calculated from terminal amino groups quantified by p-toluene sulfonic acid using thymol blue as an indicator (with respect to the polymer solution in cresol).

Example 48

Synthesis of nylon-2,6

Adiponitrile (0.216 g), ethylenediamine (0.120 g), $RuH_2(PPh_3)_4$ (0.069 g), water (0.072 g) and 1,2-dimethoxyethane (0.5 mℓ) were reacted in a sealed tube under the same conditions as Example 1. After the reaction, precipitates were separated by filtration, washed with chloroform and dried. Then, nylon-2,6 was obtained in a 99% yield. Its number average molecular weight was 3,700 which was calculated from terminal amino groups quantified by p-toluene sulfonic acid using thymol blue as an indicator. Its infrared (KBr) spectrum showed absorptions at 3,350 (N–H, s), 3,170 (N–H, s), 2,950 (C–H, s), 1,645 (C=O, s), 1,545 (N–H, m), 1,330 (m), 1,120 (m) and 800 (m) $cm^{-1}$ ; and 'H NMR spectrum ($HCO_2H$, 60MHz) : δ 1.03-1.96 (m, 4H, $-CH_2-$), 1.96-2.68 (m, 4H, $-COCH_2-$), 3.05-3.95 (m, 4H, $-N-CH_2-$) and 8.22 (br.s, 2H, –NH).

Example 49

Synthesis of nylon-3

2-aminopropionitrile (0.282 g), water (0.145 g), $RuH_2(PPh_3)_4$ (0.069 g) and 1,2-dimethoxyethane (0.5 mℓ) were reacted at 200°C in an argon gas atmosphere for 24 hours in a sealed tube. After the reaction, precipitates were separated by filtration, washed with chloroform and dried. Then, nylon-3 was obtained in a 98% yield. Its number average molecular weight was 1,600 which was calculated in the same manner as described in Example 48. Its infrared (KBr) spectrum showed absorptions at 3,290 (N–H, s), 2,940 (C–H, w), 1,640 (C=O, s), 1,545 (N–H, s), 1,435 (m), 1,115 (m) and 695 (m) $cm^{-1}$ ; and 'HNMR spectrum ($HCO_2H$, 60MHz) : δ 1.78-2.98 (m, 2H, $-COCH_2-$), 3.05-4.14 (m, 2H, $N-CH_2-$) and 7.56 (br.s, 1H, –NH).

Example 50

Synthesis of nylon-12

11-Aminoundecanenitrile (0.393 g), water (0.072 g), $RuH_2(PPh_3)_4$ (0.069 g) and 1,2-dimethoxyethane (0.5 mℓ) were reacted in an argon gas atmosphere in a sealed tube under the same conditions as Example 1. After the reaction, precipitates were separated by filtration, washed with chloroform and dried. Then, nylon-12 was obtained in a 99% yield. Its number average molecular weight was 5,000 which was calculated in the same manner as described in Example 48. Its infrared (KBr) spectrum showed absorptions at 3,290 (N-H, s), 2,940 (C-H, w), 1,640 (C=O, s), 1,545 (N-H, s), 1,435 (m), 1,115 (m) and 695 (m) $cm^{-1}$ ; and 'H NMR ($HCO_2H$, 60MHz) : δ 0.93-1.85 (m, 18H, $-CH_2-$), 1.95-2.60 (m, 2H, $-COCH_2-$), 2.82-3.43 (m, 2H, $-N-CH_2-$) and 8.45 (br.s, 2H, -NH).

Example 51

Synthesis of nylon-6T

Terephthalonitrile (0.256 g), hexamethylenediamine (0.232 g), $RuH_2(PPh_3)_4$ (0.069 g), water (0.074 g) and 1,2-dimethoxyethane (0.5 ml) were reacted at 180°C in an argon gas atmosphere for 24 hours in a sealed tube. After the reaction, precipitates were separated by filtration, washed with chloroform and dried. Then, polyhexamethyleneterephthalamide having a decomposition temperature of 265°C was obtained in a 98% yield. Its number average molecular weight was 1,200 which was calculated in the same manner as described in Example 48., Its infrared (KBr) spectrum showed absorptions at 3,160 (N-H, s), 3,070 (C-H, m), 2,920 (C-H, s), 2,860 (C-H, m), 1,620 (C=O, s), 1,535 (N-H, m), 1,410 (m), 1,285 (m) and 860 (m) $cm^{-1}$; and 'H NMR ($HCO_2H$, 60MHz) : δ 0.71-2.34 (m, 8H, $-CH_2-$), 2.90-3.76 (m, 4H, $-NCH_2$), 6.21 (br.s, 2H, NH) and 7.00-7.54 (m, 4H, ArH).

Example 52

Synthesis of poly-p-cyclohexaneadipamide

Adiponitrile (0.216 g), 1,4-cyclohexanediamine (0.228 g), $RuH_2(PPh_3)_4$ (0.069 g), water (0.074 g) and 1,2-dimethoxyethane (0.5 mℓ) were reacted at 180°C in an argon gas atmosphere for 24 hours in a sealed tube. After the reaction, precipitates were separated by filtration washed with chloroform and dried. Then, polyamide having a decomposition temperature of 208°C was obtained in a 98% yield. Its number average molecular weight was 1,000 which was calculated in the same manner as described in Example 48. Its infrared (KBr) spectrum showed absorptions at 3,180 (N-H, s), 2,925 (C-H, s), 2,860 (C-H, m), 1,630 (C=O, s), 1,540 (N-H, s), 1,410 (m), 1,115 (m) and 745 (m) $cm^{-1}$ ; and 'H NMR ($HCO_2H$, 60mHz) : δ 0.84-1.97 (m, 16H), 2.70-3.27 (m, 2H, -NCH'-) and 6.76 (br.s, 2H, NH).

Example 53

Synthesis of Polypiperazineadipamide

Adiponitrile (0.216 g), piperazine (0.172 g), $RuH_2(PPh_3)_4$ (0.069 g), water (0.077 g) and 1,2-dimethoxyethane (0.5 mℓ) were reacted at 180°C in an argon gas atmosphere for 24 hours in a sealed tube. After the reaction, precipitates were separated by filtration, washed with chloroform and dried. Then, polyamide having a decomposition temperature of 230°C was obtained in a 98% yield. Its number average molecular weight was 2,200 which was calculated in the same manner as described in Example 48. Its IR(KBr) spectrum showed absorptions at 2,930 (C-H, S), 2,870 (C-H, S), 1,635 (C=O, S), 1,435 (S), 1,250 (m), 1,205 (m) and 1,015 (m) $cm^{-1}$ ; and 'H NMR ($HCO_2H$, 60mHz) : δ 0.72-1.46 (m, 4H, -CH2-), 1.52-2.37 (m, 4H, $-CH_2CO-$) and 2.70-3.69 (m, 8H, $-NCH_2-$).

Example 54

Synthesis of polyhexamethylene-p-phenylenediacetamide

1,4-Phenylenediacetonitri1e (0.312 g), hexamethylenediamine (0.232 g), $RuH_2(PPh_3)_4$ (0.069 g), water (0.074 g) and 1,2-dimethoxyethane (0.5 mℓ) were reacted at 180°C in an argon gas atmosphere for 24 hours in a sealed tube. After the reaction, precipitates were separated by filtration, washed with chloroform and dried. A polyamide that did not melt at 300°C was obtained in a 93% yield. Its number average molecular weight was 14,000, which was calculated in the same manner as described in Example 48. Its IR(KBr) spectrum showed absorptions at 3,250 (N-H, m), 2,920 (C-H, s), 2,850 (C-H, m), 1,630 (C=O, s), 1,530 (N-H, m), 1,425 (m) and 740 (m) $cm^{-1}$.

As is clear from the foregoing Examples, the use of ruthenium, rhodium and molybdenum complexes

according to the present invention enables amides to be efficiently prepared directly from nitriles, amines and water, in a single-step process, so that curtailment of reaction time, compaction of equipments and clean operations can be realised as compared with the conventional two-step process for amide synthesis. In particular, the advantage of the present invention lies in the fact that the reaction can be effected under neutral conditions with a small amount of water, which is economical in energy. Further, by using dinitriles and diamines, or aminonitriles according to the present invention, polyamides can be produced in a single-step manufacturing process.

## Claims

1. A process for the preparation of organic amides by reaction of a carbonitrile and a primary or secondary amine in which the carbonitrile is reacted directly with an equivalent amount of the primary or secondary amine, the reaction taking place by heating the reactants in the presence of at least a stoichiometric amount of water relative to the carbonitrile reactant, and in the presence of a catalyst comprising a complex containing one or more of ruthenium, rhodium, and molybdenum.

2. A process according to claim 1, in which the catalyst is a ruthenium complex.

3. A process according to claim 2, in which the catalyst is of the formula $RuH_2$ $(PPh_3)_4$ or $RuH_2$ $(CO)(PPh_3)_3$.

4. A process according to claim 1, in which the catalyst is a rhodium complex.

5. A process according to claim 4, in which the amide is N-butylacetamide and the catalyst is of the formula Rh (CO) (OH) $(PPh_3)_2$.

6. A process according to claim 1, in which the catalyst is a molybdenum complex.

7. A process according to claim 6, in which the catalyst is of the formula Mo $(CO)_6$.

8. A process according to any one of the preceding claims, in which the amount of catalyst is from 0.001 to 10 mol % based on the carbonitrile.

9. A process according to claim 8, in which the amount of catalyst is from 0.1 to 3 mol % based on the carbonitrile.

10. A process according to any one of the preceding claims carried out under an inert gas atmosphere.

11. A process according to any one of the preceding claims, in which the reaction is carried out in the presence of 1,2-dimethoxyethane, dioxane, pryidine, diglyme and/or tetrahydrofuran as solvent.

12. A process according to any one of the preceding claims, in which the reaction mixture is heated to a temperature not higher than 250°C.

13. A process according to any one of the preceding claims, in which the reaction mixture contains from 1-100 equivalents of water relative to the amount of carbonitrile.

14. A process according to claim 13, in which mixture contains from 1-3 equivalents of water relative to the amount of carbonitrile.

15. A process according to any one of claims 1 to 14, in which the carbonitrile reactant is of the formula $R^1CN$ where $R^1$ denotes (i) an alkyl, alkenyl, alkynyl, cycloalkyl or aryl group of up to 20 carbon atoms, or (ii) a univalent residue of a 3 to 7 membered heterocyclic group having in the ring up to 3 hetero atoms each of which is O, N or S, with the proviso that the groups defined for $R^1$ optionally contain one or more of the following substituents : an aryl, alkenyl or alkynyl group of up to 12 carbon atoms, a univalent residue of a 3 to 7 membered heterocyclic group containing up to 3 hetero atoms each of which is O, N or S, or an OR, $CO_2R$, $NR_2$, SR, $SiR_3$ or $CONR_2$ group where R is an optionally substituted alkyl group containing up to 10 carbon atoms or a phenyl group ; and the amine reactant is of the formula $R^2R^3NH$ where each of $R^2$ and $R^3$ represents a hydrogen atom or a group as defined for $R^1$, or $-NR^2R^3$ is a saturated or unsaturated heterocyclic ring optionally containing O, S or another N as hetero atom.

16. A process according to any one of claims 1 to 14, in which the carbonitrile is of the formula $R^4(CN)_2$ where $R^4$ denotes (i) an alkylene, alkenylene, alkynylene, cycloalkyblene or arylene group having up to 20 carbon atoms ; (ii) a bivalent residue of a 3 to 7 membered heterocyclic group having in the ring up to 3 hetero atoms each of which is O, N or S ; or (iii) a group consisting of two aliphatic hydrocarbon residues each having up to 10 carbon atoms bridged by phenylene group, an O, N or S hetero atom, or a bivalent residue of a 3 to 7 membered heterocyclic group having in the ring up to 3 hetero atoms each of which is O, N or S ; with the proviso that any of such groups optionally contain one or more of the following substituents : an aryl, alkenyl or alkynyl group of up to 12 carbon atoms, a univalent residue of a 3 to 7 membered heterocyclic group containing up to 3 hetero atoms each of which is O, N or S, or an OR, $CO_2R$, $NR_2$, SR, $SiR_3$ or $CONR_2$ group where R is an optionally substituted alkyl group containing up to 10 carbon atoms or a phenyl group ; and the amine is a diamine of the formula $HN(R^5) - R^6 - (R^7)NH$ where each of $R^5$ and $R^7$

represents a hydrogen atom or a group as defined for $R^1$, and $R^6$ is a group as defined for $R^4$.

17. A process according to claim 16, in which the nitrile reactant is an alkylenedinitrile of the formula $NC(CH_2)_n(CN)$ where n is an integer of 1-20, and the diamine reactant is an alkylenediamine of the formula $HN(R^5)-(CH_2)_m-NHR^7$ where m is an integer of 1-20.

18. A process according to any one of claims 1 to 4, in which the carbonitrile reactant and the amine reactant are both compounds of the formula $HN(R^5)-R^6-CN$ where $R^5$ and $R^6$ are as defined in claim 15.

19. A process according to claim 18, in which the nitrile reactant and the amine reactant are both compounds of the formula $NC(CH_2)_pNHR^5$ where p is an integer of 1-3 or 5-20.

## Ansprüche

1. Verfahren zur Herstellung von organischen Amiden durch Umsetzung eines Carbonitrils und eines primären oder sekundären Amins, bei dem das Carbonitril direkt mit einer äquivalenten Menge des primären oder sekundären Amins umgesetzt wird, die Umsetzung durch Erhitzen der Reaktionspartner in Gegenwart wenigstens einer stöchiometrischen Menge an Wasser in Bezug auf den Carbonitril-Reaktionspartner und in Gegenwart eines Katalysators stattfindet, der einen Komplex umfaßt, welcher eines oder mehrere der Elemente Ruthenium, Rhodium und Molybdän enthält.

2. Verfahren nach Anspruch 1, in welchem der Katalysator ein Rutheniumkomplex ist.

3. Verfahren nach Anspruch 2, in welchem der Katalysator die Formel $RuH_2\ (PPh_3)_4$oder $RuH_2\ (CO)PPh_3)_3$ hat.

4. Verfahren nach Anspruch 1, in dem der Katalysator ein Rhodiumkomplex ist.

5. Verfahren nach Anspruch 4, in dem das Amid N-Butylacetamid ist und der Katalysator die Formel $Rh(CO)(OH)(PPh_3)_2$ hat.

6. Verfahren nach Anspruch 1, in dem der Katalysator ein Molybdänkomplex ist.

7. Verfahren nach Anspruch 6, in dem der Katalysator die Formel $Mo(CO)_6$ hat.

8. Verfahren nach einem der vorausgehenden Ansprüche, in dem die Katalysatormenge 0,001 bis 10 Mol-%, bezogen auf das Carbonitril, beträgt.

9. Verfahren nach Anspruch 8, in dem die Katalystormenge 0,1 bis 3 Mol-%, bezogen auf das Carbonitril, beträgt.

10. Verfahren nach einem der vorausgehenden Ansprüche, das unter einer Inertgasatmosphäre durchgeführt wird.

11. Verfahren nach einem der vorausgehenden Ansprüche, in welchem die Umsetzung in Gegenwart von 1,2-Dimethoxyethan, Dioxan, Pyridin, Diglyme und/oder Tetrahydrofuran als Lösungsmittel durchgeführt wird.

12. Verfahren nach einem der vorausgehenden Ansprüche, in dem das Reaktionsgemisch auf eine Temperatur nicht höher als 250°C erhitzt wird.

13. Verfahren nach einem der vorausgehenden Ansprüche, in dem das Reaktionsgemisch 1 bis 100 Äquivalente Wasser in Bezug auf die Carbonitrilmenge enthält.

14. Verfahren nach Anspruch 13, in dem das Gemisch 1 bis 3 Äquivalente Wasser in Bezug auf die Carbonitrilmenge enthält.

15. Verfahren nach einem der Ansprüche 1 bis 14, in dem der Carbonitril-Reaktionspartner die Formel $R^1CN$ hat, worin $R^1$ (i) eine Alkyl-, Alkenyl-, Alkinyl-, Cycloalkyl- oder Arylgruppe mit bis zu 20 Kohlenstoffatomen oder (ii) einen einbindigen Rest einer 3- bis 7-gliedrigen heterozyklischen Gruppe mit bis zu 3 Heteroatomen im Ring, von denen jedes O, N oder S ist, bedeutet, wobei die durch $R^1$ definierte Gruppe gegebenenfalls einen oder mehrere der folgenden Substituenten enthält : eine Aryl-, Alkenyl- oder Alkinylgrupe mit bis zu 12 Kohlenstoffatomen, einen einbindigen Rest einer 3- bis 7-gliedrigen heterozyklischen Gruppe mit bis zu 3 Heteroatomen, von denen jedes 0, N oder S ist, oder eine Gruppe OR,$CO_2R$, $NR_2$, SR, $SiR_3$ oder $CONR_2$, worin R eine gegebenenfalls substituierte Alkylgruppe mit einem Gehalt von bis zu 10 Kohlenstoffatomen oder eine Phenylgruppe ist ; und der AminReaktionspartner die Formel $R^2R^3NH$ hat, worin $R^2$ und $R^3$ jeweils ein Wasserstoffatom oder eine wie oben für $R^1$ definierte Gruppe bedeuten oder $-NR^2R^3$ ein gesättigter oder ungesättigter heterozyklischer Ring ist, der als Heteroatom gegebenenfalls O, S oder ein weiteres N enthält.

16. Verfahren nach einem der Ansprüche 1 bis 14, in dem das Carbonitril die Formel $R^4(CN)_2$ hat, worin $R^4$ (i) eine Alkylen-, Alkenylen-, Alkinylen-, Cycloalkylen- oder Arylengruppe mit bis zu 20 Kohlenstoffatomen, (ii) einen zweibindigen Rest einer 3- bis 7-gliedrigen heterozyklischen Gruppe mit bis zu 2 Heteroatomen im Ring, von denen jedes O, N oder S ist, oder (iii) eine Gruppe bedeutet, die aus zwei aliphatischen jeweils bis zu 10 Kohlenstoffatomen enthaltenden, durch eine Phenylengruppe, ein O–, N– oder S-Heteroatom oder einen zweibindigen Rest einer 3- bis 7gliedrigen heterozyklischen Gruppe mit bis zu 3 Heteroatomen im Ring, von denen jedes O, N oder S ist, verbrückten Kohlenwasserstoffresten besteht, wobei jede dieser Gruppen gegebenenfalls einen oder mehrere der folgenden Substituenten enthält : eine Aryl-, Alkenyl- oder Alkinylgruppe mit bis zu 12 Kohlenstoffatomen, einen einbindigen Rest einer 3- bis 7-gliedrigen heterozyklischen Gruppe mit bis zu 3 Heteroatomen, von denen jedes O, N oder S ist, oder eine Gruppe OR, $CO_2R$, $NR_2$, SR, $SiR_3$ oder $CONR_2$, worin R eine gegebenenfalls substituierte Alkylgruppe mit bis zu 10 Kohlenstoffatomen oder eine Phenylgruppe ist ; und das Amin ein Diamin der Formel $HN(R^5)-R^6-(R^7)NH$ ist, worin jede der Gruppen $R^5$ und $R^7$ ein Wasserstoffatom oder eine wie für $R^1$ definierte Gruppe bedeutet und $R^6$ wie für $R^4$ definierte Gruppe ist.

17. Verfahren nach Anspruch 16, in dem der Nitril-Reaktionspartner ein Alkylendinitril der Formel NC $(CH_2)_n(CN)$ ist, worin n eine ganze Zahl von 1 bis 20 bedeutet, und der Diamin-Reaktionspartner ein Alkylendiamin der Formel HN $(R^5)$ $-(CH_2)_n-NHR^7$ ist, worin m eine ganze Zahl von 1 bis 20 ist.

18. Verfahren nach einem der Ansprüche 1 bis 4, in dem der Carbonitril-Reaktionspartner und der Amin-Reaktionspartner beide Verbindungen der Formel $HN(R^5)-R^6-CN$ sind, worin $R^5$ und $R^6$ wie oben in Anspruch 15 definiert sind.

19. Verfahren nach Anspruch 18, in dem der Nitril-Reaktionspartner und der Amin-Reaktionspartner beide Verbindungen der Formel $NC(CH_2)_pNHR^5$ sind, worin p eine ganze Zahl von 1 bis 3 oder 5 bis 20 ist.

## Revendications

1. Procédé pour la préparation d'amides organiques, par la réaction d'un carbonitrile et d'une amine primaire ou secondaire, procédé dans lequel le carbonitrile est mis à réagir directement avec une quantité équivalente de l'amine primaire ou secondaire, la réaction étant mise en oeuvre par chauffage des réactifs en présence d'au moins une quantité d'eau stoechiométrique par rapport au réactif carbonitrile, et en présence d'un catalyseur comprenant un complexe contenant au moins l'un des éléments ruthénium, rhodium et molybdène.

2. Procédé selon la revendication 1, dans lequel le catalyseur est un complexe de ruthénium.

3. Procédé selon la revendication 2, dans lequel le catalyseur a la formule $RuH_2(PPh_3)_4$ ou $RuH_2(CO)(PPh_3)_3$.

4. Procédé selon la revendication 1, dans lequel le catalyseur est un complexe du rhodium.

5. Procédé selon la revendication 4, dans lequel l'amide est le N-butylacétamide et le catalyseur a la formule $Rh(CO)-(OH)(PPh_3)_2$.

6. Procédé selon la revendication 1, dans lequel le catalyseur est un complexe du molybdène.

7. Procédé selon la revendication 6, dans lequel le catalyseur a la formule $Mo(CO)_6$.

8. Procédé selon l'une quelconque des revendications précédentes, dans lequel la quantité de catalyseur est de 0,001 à 10% en moles par rapport au carbonitrile.

9. Procédé selon la revendication 8, dans lequel la quantité de catalyseur est de 0,1 à 3% en moles par rapport au carbonitrile.

10. Procédé selon l'une quelconque des revendications précédentes, mis en oeuvre sous une atmosphère d'un gaz inerte.

11. Procédé selon l'une quelconque des revendications précédentes, dans,lequel la réaction est mise en oeuvre en présence de 1,2-diméthoxyéthane, de dioxanne, de pyridine, de diglyme et/ou de tétrahydrofuranne servant de solvant.

12. Procédé selon l'une quelconque des revendications précédentes, dans lequel le mélange réactionnel est chauffé à une température non-supérieure à 250°C.

13. Procédé selon l'une quelconque des revendications précédentes, dans lequel le mélange réactionnel contient de 1100 équivalents d'eau par rapport à la quantité de carbonitrile.

14. Procédé selon la revendication 13, dans lequel le mélange contient de 1-3 équivalents d'eau par rapport à la quantité de carbonitrile.

15. Procédé selon l'une quelconque des revendications 1 à 14, dans lequel le réactif carbonitrile a la formule $R^1CN$, où $R^1$ désigne (i) un groupe alkyle, alcényle, alcynyle, cycloalkyle ou aryle ayant jusqu'à 20 atomes de carbone, ou (ii) un résidu monovalent d'un groupe hétérocyclique à 3-7 chaînons ayant dans le cycle jusqu'à 3 hétéroatomes, dont chacun est O, N ou S, à la condition que les groupes définis par $R^1$ contiennent facultativement un ou plusieurs des substituants suivants : un groupe aryle, alcényle ou alcynyle ayant jusqu'à 12 atomes de carbone, un résidu monovalent d'un groupe hétérocyclique à 3-7 chaînons contenant jusqu'à 3 hétéroatomes, dont chacun est O, N ou S, ou encore un groupe OR, $CO_2R$, $NR_2$, SR, $SiR_3$ ou $CONR_2$, où R est un groupe alkyle facultativement substitué ayant jusqu'à 10 atomes de carbone,ou

un groupe phényle ; et le réactif amine a la formule $R^2R^3NH$, dans laquelle chacun des radicaux $R^2$ et $R^3$ représente un atome d'hydrogène ou un groupe tel que défini pour $R^1$, ou encore $-NR^2R^3$ est un noyau hétérocyclique saturé ou insaturé contenant facultativement en tant qu'hétéroatome O, S ou un autre atome N.

16. Procédé selon l'une quelconque des revendications 1 à 14, dans lequel le carbonitrile a la formule $R^4(CN)_2$, où $R^4$ désigne (i) un groupe alkylène, alcénylène, alcynylène, cycloalcylène ou arylène ayant jusqu'à 20 atomes de carbone ; (ii) un résidu bivalent d'un groupe hétérocyclique à 3-7 chaînons ayant dans le cycle jusqu'à 3 hétéroatomes dont chacun est O, N ou S ; et (iii) un groupe constitué de deux résidus hydrocarbonés aliphatiques ayant chacun jusqu'à 10 atomes de carbone, pontés par un groupe phénylène, un hétéroatome O, N ou S ou un résidu bivalent d'un groupe hétérocyclique à 3-7 chaînons ayant dans le cycle jusqu'à 3 hétéroatomes dont chacun est O, N ou S ; à la condition que l'un quelconque de ces groupes contienne facultativement un ou plusieurs des substituants suivants : un groupe aryle, alcényle ou alcynyle ayant jusqu'à 12 atomes de carbone, un résidu monovalent d'un groupe hétérocyclique à 3-7 chaînons contenant jusqu'à 3 hétéroatomes dont chacun est O, N ou S, et un groupe OR, $CO_2R$, $NR_2$, SR, $SiR_3$ ou $CONR_2$, où R est un groupe alkyle éventuellement substitué contenant jusqu'à 10 atomes de carbone, ou un groupe phényle ; et l'amine est une diamine de formule $HN(R^5)R^6(R^7)NH$, dans laquelle chacun des radicaux $R^5$ et $R^7$ représente un atome d'hydrogène ou un groupe tel que défini pour $R^1$, et $R^6$ est un groupe tel que défini pour $R^4$.

17. Procédé selon la revendication 16, dans lequel le réactif nitrile est un alkylènedinitrile de formule $NC(CH_2)_n-(CN)$, dans laquelle n est un entier de 1 à 20, et le réactif diamine est une alkylènediamine de formule $HN(R^5)(CH_2)_mNHR^7q$, où est un entier de 1 à 20.

18. Procédé selon l'une quelconque des revendications 1 à 4, dans lequel le réactif carbonitrile et le réactif amine sont tous les deux des composés de formule $HN(R^5)R^6CN$ dans laquelle $R^5$ et $R^6$ sont tels que définis dans la revendication 15.

19. Procédé selon la revendication 18, dans lequel le réactif nitrile et le réactif amine sont tous les deux des composés de formule $NC(CH_2)_pNHR^5$, dans laquelle p est un entier de 1 à 3 ou de 5 à 20.